# EUROPEAN PATENT SPECIFICATION

(11) **EP 1 707 142 B1**
(45) Date of publication and mention of the grant of the patent: **10.03.2010**
(21) Application number: 06251836.0
(22) Date of filing: 31.03.2006
(51) Int. Cl.: A61B 17/80

(54) **Metatarsal fixation plate**
Mittelfuss-Fixationsplatte
Plaque de fixation métatarsienne

(30) Priority: 31.03.2005 US 94954
(43) Date of publication of application: 04.10.2006
(73) Proprietor: DePuy Products, Inc., Warsaw, IN 46581 (US)
(72) Inventor: Sanders, Roy, Tampa FL 33606 (US); Prasad, Priya, Warsaw IN 46580 (US); Bremer, Chris, Warsaw IN 46582 (US); Myerson, Mark, Baltimore MD21202 (US)
(74) Representative: Gover, Richard Paul

(56) References cited:
- EP-A- 1 452 145
- EP-A- 1 616 528
- US-A- 4 503 848
- US-A- 5 681 311
- US-A1- 2004 092 935
- ANONYMOUS: "Dimpled plate" RESEARCH DISCLOSURE, MASON PUBLICATIONS, HAMPSHIRE, GB, vol. 476, no. 64, December 2003 (2003-12), XP007133232 ISSN: 0374-4353

## Description

The present invention relates to a plate for use in fixation of the metatarsal bones of the foot for fracture reduction, hallux valgus correction and similar surgical procedures.

A hallux valgus deformity is a very common foot disorder, often referred to as a bunion. The most common cause attributed to this deformity is improper or ill-fitting footwear, although other biomechanical, traumatic and metabolic factors may actually be more likely etiologies for this condition. The hallux valgus deformity manifests itself as a medial deviation of the first metatarsal, and a lateral deviation and/or rotation of the hallux. This condition can lead to painful motion of the joint, difficulty in wearing shoes, and adjustments in gait that may ultimately cause problems farther up the leg. In minor cases, orthotics may alleviate, but not correct, the deformity.

Surgical treatment of a hallux valgus deformity should preferably relieve symptoms and pain, restore function and correct or reduce the deformity. For some indications, the surgical treatment is limited to removal of a medial portion of the metatarsus to reduce the prominence of the bunion. In more severe cases, or for elderly patients, resectional arthroplasty of the metatarso-phalangeal (MTP) joint, with or without implant, or joint arthrodesis may be indicated, although these procedures result in loss of motion at that joint.

In the majority of hallux valgus cases, the surgical treatment involves an osteotomy to correct the structural deformities associated with the cuneiform, metatarsal and phalanges. The osteotomy is often accompanied by a lateral release and capsulorraphy to address adaptive changes of the associated cartilage and ligaments. The goal is to return the metatarsus and MTP joint to their optimum anatomic positions and restore acceptable hallux valgus, inter-metatarsal angle and distal metatarsal angles. While phalanx and cuneiform osteotomies have been implemented, the prevalent procedure involves removing a wedge from the distal or head end of the metatarsus. Chevron osteotomies are typically used for correction of mild deformities, while Mitchell's transverse osteotomies are used for moderate deformities. The more severe cases usually require a Scarf osteotomy in which a Z-shaped osteotomy in the transverse plane extends from the metatarsal head to the base. Depending upon the nature of the deformity, the osteotomy may involve removal of bone (closing wedge osteotomy) or insertion of a wedge of bone (opening wedge osteotomy).

With all of these osteotomies, solid fixation of the bone segments is essential to achieve complete union. The same is true for reduction of a fracture of the metatarsus. Metatarsal fractures are relatively common conditions and range in severity from an incomplete separation, to complete separation with displacement, to severe comminution.

In the past, one typical surgical approach to correcting metatarsal fractures has been to fix the bone segments using bone screws passing from segment to segment. Other approaches include the use of cerclage wire, internal medullary pinning with K-wire, and mono-filament wire fixation. In some cases, a flat plate has been mounted to the bone bridging the fracture or osteotomy opening. These flat plates are typically limited to providing axial compression for simple fractures of the metatarsal long bone.

US-4503848 discloses an osteosynthesis plate allowing for immobilisation of pieces of bone. The longitudinal edges of the osteosynthesis plate diverge in the manner of a trapezoid. The plate is provided with two adjacent rows of slots for its screws.

An anonymous research disclosure entitled "Dimpled plate", research disclosure, Mason publications, Hampshire, GB, volume 476, no. 64, December 2003 discloses a resorbable bone plate. The bone plate include multiple countersunk potential screw hole locations. The screw hole locations are indicated by surface dimples. The dimples obviate the need for predrilled holes.

There is a need for a fixation device that can be used for any surgical treatment of the metatarsus, especially of the first metatarsus. The need extends further to a fixation device that accurately reduces the fracture or osteotomy and maintains the reduction until union occurs.

The scope of the present invention is defined by the appended claims.

To address the need for a fixation device for use in reducing the metatarsus, embodiments of the present invention provide a fixation plate that is sized and configured to generally conform to the shaft of the metatarsus between the head and base. The plate includes a head end that is wider than the base end, with curved side edges that gradually taper between the two ends. In one feature of the invention, the side edges are curved to produce a necked-down portion between the two end edges that has a width less than the width of either end. This contour reduces the overall profile of the plate.

The profile of the plate is preferably further reduced by curving the plate across its width and along the entire length of the plate. The curvature corresponds to the shape of the shaft of the metatarsus. Another feature to reduce the prominence of the plate is the minimal thickness of the plate, about 0.9 mm in the preferred embodiment.

For embodiments of the invention, the plate is provided with a plurality of screw holes staggered across the length and width of the plate. In the preferred embodiment, twelve screw holes are provided so that the surgeon may select a number, combination and location of bone screws to optimally secure the plate to the bone. Moreover, the array of screw holes provided by the plate of the present invention allows the surgeon to position the screws where they are most needed to reduce a fracture or wedge osteotomy.

The screw holes may be staggered to avoid conflict of the screws within the bone. In another aspect, the screw holes include a circumferential chamfer to accommodate bone screws of different sizes.

One benefit of the fixation device of embodiments of the present invention is that it provides the surgeon with great flexibility in screw placement to reduce a fracture and maintain this reduction until union occurs. Another benefit of certain embodiments is that the plate has a minimal prominence or profile above the bone, which reduces soft tissue trauma and minimizes the ability to visibly detect the presence of the plate in the patient's toe.

Embodiments of the invention will now be described by way of example with reference to the accompanying drawings, in which:
FIG. 1 is an enlarged view of the first ray of the foot, including the metatarsus and phalanx, with a fixation plate in accordance with one embodiment of the invention mounted thereon.
FIG. 2 is a top elevational view of the fixation plate shown in FIG. 1.
FIG. 3 is an end elevational view of the fixation plate of FIG. 2.
FIG. 4 is a side elevational view of the fixation plate of FIG. 2. Referring to the drawings, FIG. 1 shows the first ray of a foot, having a plate 10 mounted on it. The first ray includes the cuneiform, the metatarsus and the phalanges. The plate 10 is configured for reducing fractures or bone segments of the shaft metatarsus, preferably between the base and the head of that bone.

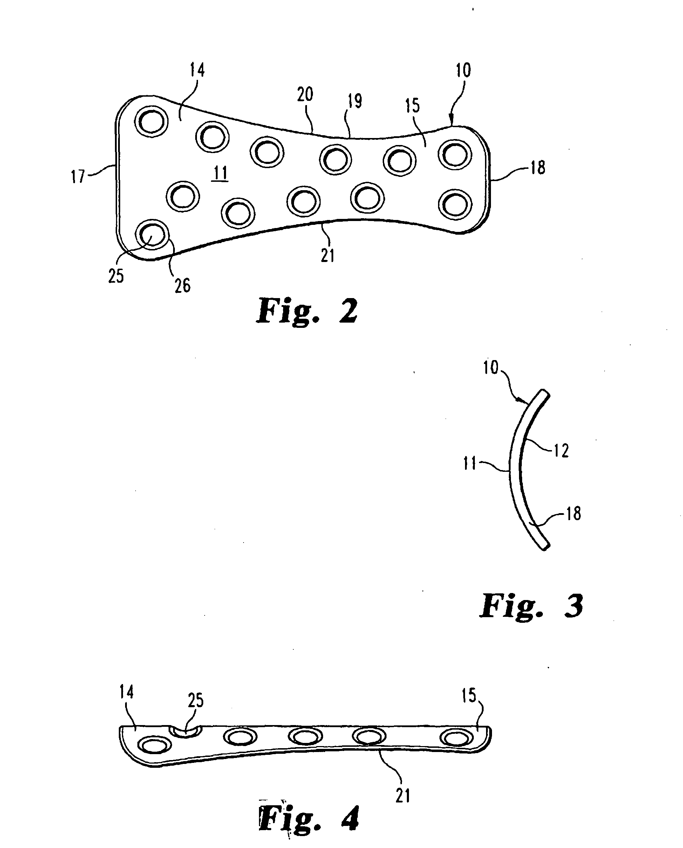

FIGS. 2 to 4 show more specific details of the plate 10, including an upper surface 11 and a lower surface 12 that is configured for substantially flush mounting onto the shaft of the metatarsus. In particular, the lower surface 12 is formed at a radius that approximates the radius of the metatarsus. In a specific embodiment, the radius is about 15.0 mm. In order to further reduce the prominence of the plate above the bone, the plate has a substantially uniform thickness between the upper and lower surfaces 11, 12 that is minimized without compromising strength of the plate. It has been found in a specific embodiment that a thickness of less than 1.0 mm, and preferably about 0.9 mm, provides a minimal profile above the bone while retaining sufficient strength to reduce and maintain the reduction for union to occur in the bone. The curvature of the plate (as shown best in FIG. 3) increases the bending stiffness of the plate. In the preferred embodiment, the plate is formed of a biocompatible metal, such as titanium. In a specific embodiment, the plate is formed of a titanium alloy, such as Ti-6Al-4V.

The plate 10 includes a head end 14 that is wider than the base end 15, to generally correspond to the geometry of the metatarsus which is narrower at its base than at its head. In a preferred embodiment, the head edge 17 has a width of about 21.0 mm while the base edge 18 has a width of about 14.0 mm.

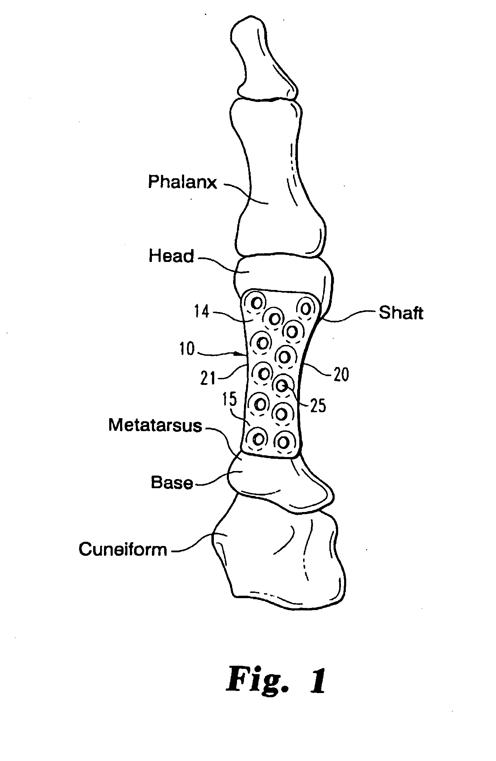

The prominence of the plate 10 between the two end edges 17, 18 is minimized by curving the side edges 20, 21. In particular, the edges are curved at a large radius so that the plate includes a necked-down region 19 that has a width less than the width of the base end edge 18. The side edges are curved at different radii to conform more closely to the geometry of the shaft of the metatarsus. In a specific embodiment, the side edge 20, which is the medial edge in FIG. 1, is curved at a radius of about 43.0 mm. The opposite side edge 21, or the lateral edge, is curved at a radius that is more than twice the radius of the medial edge. In a specific embodiment, the lateral edge 21 is curved at a radius of about 90.0 mm. The two curved side edges result in a plate width at the necked-down region of about 12.0 mm.

As can be seen in FIG. 2, the corners of the plate between end edges 17, 18 and side edges 20, 21 are curved, preferably at a radius of about 3.8 mm. In addition, all of the edges of the plate 10 are curved across the thickness between the upper and lower surfaces 11, 12. These rounded corners and edges reduce the trauma to soft tissue surrounding the metatarsus.

In accordance with one feature of the invention, the plate 10 defines a plurality of holes 25 therethrough that are configured to receive a bone engaging fastener, such as a bone screw. The holes may be configured to receive a non-locking or a self-locking screw. In the latter instance, the holes 25 may be tapered at a conventional angle (for example about 12°) that is acceptable for a locking screw.

In the preferred embodiment, each hole 25 includes a circumferential chamfer 26 that allows the hole to accommodate differently sized screws. In a specific embodiment, the holes 25 may accept 2.7 mm or 3.5 mm locking or non-locking cortical screws to secure the plate 10 to the bone.

In a further feature of the plate 10, the screw holes 25 are staggered throughout the entire width and length of the plate. This pattern of screw holes provides significant advantages. One advantage is that the plate can accommodate several screw holes to increase the versatility of the plate. In the preferred embodiment, a plate 10 for the first metatarsal includes twelve screw holes from which the surgeon may select. If the deformity being treated is a fracture of the metatarsus, the surgeon may select among several combinations of two or more screws on either side of the fracture. If the metatarsus includes multiple fractures, the multiple screw holes virtually ensure that every bone segment can be reduced and fixed until union occurs. If a wedge osteotomy at the head of the metatarsus is being reduced, the screws may be placed at the pair of screw holes at the base end 15 of the plate and any number of screw positions may be selected at the head end 14 depending upon the position and type of osteotomy.

Another advantage of the arrangement of the screw holes 25 on plate 10 is that no two screws will conflict within the metatarsus bone, even if a bone screw is driven through every screw hole 25. While the curvature of the plate produces the benefits discussed above, it creates the risk of conflict between adjacent screws. Staggering the screw holes as shown in FIG. 2 eliminates the possibility of such conflicts. In a preferred embodiment of the invention, the pairs of screws at the head end 14 and base end 15 may be transversely aligned because the pairs of holes are separated by a sufficient width to avoid contact within the bone. Of the remaining screws along the interior length of the plate 10 the screw holes adjacent the medial edge 20 are preferably staggered by about 3.0 mm relative to the screw holes adjacent to the lateral edge 21.

## Claims

1. A fixation device for reduction of bone segments of the metatarsus comprising:
an elongated plate (10) having a head end (14) and a base end (15), said plate (10) having a width at said head end (14) that is greater than the width at said base end (15);
said plate (10) further including opposite curved side edges (20, 21) between said head and base ends (14, 15), said side edges (20, 21) curved to produce a necked-down portion (19) of said plate (10) between said head and base ends (14, 15), wherein said necked-down portion (19) has a width less than the width of said base end (15); and
said plate (10) further defining a pair of holes (25) at each of said head end (14) and said base end (15) and a plurality of holes (25) between said pairs of holes (25);
wherein one of said side edges (20, 21) is curved at a first radius and the opposite one of said side edges (20, 21) is curved at a second radius greater than said first radius.

2. The fixation device of claim 1, wherein said plurality of holes (25) are staggered relative to each other.

3. The fixation device of claim 2, wherein said plurality of holes (25) include a first group of holes (25) adjacent one of said side edges (20, 21) and a second group of holes (25) adjacent the opposite one of said side edges (20, 21) and staggered relative to said first group of holes (25).

4. The fixation device of claim 3, wherein said first and second groups of holes (25) each includes at least two holes (25).

5. The fixation device of claim 1, wherein said elongated plate (10) is curved between said side edges (20, 21) substantially uniformly along the length of said plate (10).

6. The fixation device of claim 1, wherein at least some of said holes (25) includes a circumferential chamfer (26) configured so that said hole (25) accepts differently sized screws therethrough.

7. The fixation device of claim 1, wherein said plate (10) has a thickness that is less than about 1 mm.

## Patentansprüche

1. Fixiervorrichtung für die Reduktion von Knochensegmenten des Mittelfußes, die auf weist:
eine längliche Platte (10) mit einem Kopfende (14) und einem Basisende (15), wobei die Platte an dem Kopfende (14) eine Breite hat, die größer ist als die Breite an dem Basisende (15);
wobei die Platte (10) weiter gegenüber liegende gebogene Seitenkanten (20, 21) zwischen dem Kopf- und dem Basisende (14, 15) umfasst, wobei die Seitenkanten (20, 21) so gekrümmt sind, dass sie einen halsartigen Bereich (19) der Platte (10) zwischen dem Kopf und dem Basisende (14, 15) erzeugen, wobei der halsartige Abschnitt (19) eine Breite hat, die kleiner ist als die Breite des Basisendes (15); und
wobei die Platte (10) weiter ein Paar Löcher (25) sowohl an dem Kopfende (14) als auch an dem Basisende (15) und eine Vielzahl von Löchern (25) zwischen dem Paar der Löcher (25) definiert;
wobei eine der Seitenkanten (20, 21) mit einem ersten Radius gekrümmt ist und die gegenüber liegende der Seitenkanten (20, 21) mit einem zweiten Radius, der größer als der erste Radius ist, gekrümmt ist.

2. Fixiervorrichtung nach Anspruch 1, bei der die Vielzahl der Löcher (25) in Bezug auf einander abgestuft sind.

3. Fixiervorrichtung nach Anspruch 2, bei der die Vielzahl der Löcher (25) eine erste Gruppe Löcher (25) benachbart einer der Seitenkanten (20, 21) und eine zweite Gruppe Löcher (25) benachbart der gegenüberliegenden der Seitenkanten (20, 21) und in Bezug auf die erste Gruppe Löcher (25) abgestuft umfasst.

4. Fixiervorrichtung nach Anspruch 3, bei der die erste und die zweite Gruppe Löcher (25) jeweils wenigstens zwei Löcher (25) umfasst.

5. Fixiervorrichtung nach Anspruch 1, bei der die längliche Platte (10) zwischen den Seitenkanten (20, 21) im Wesentlichen gleichförmig über die Länge der Platte (10) gekrümmt ist.

6. Fixiervorrichtung nach Anspruch 1, bei der wenigstens einige der Löcher (25) eine Abschrägung (26) am Umfang umfassen, die so gestaltet ist, dass das Loch (25) unterschiedlich große Schrauben aufnimmt.

7. Fixiervorrichtung nach Anspruch 1, bei der die Platte (10) eine Dicke hat, die kleiner als etwa 1 mm ist.

## Revendications

1. Dispositif de fixation destiné à une réduction de segments d'os du métatarse comprenant :
→ une plaque allongée (10) qui présente une extrémité de tête (14) et une extrémité de base (15), ladite plaque (10) présentant une largeur au niveau de ladite extrémité de tête (14) qui est plus grande que la largeur au niveau de ladite extrémité de base (15) ;
→ ladite plaque (10) comprenant en outre des bords latéraux incurvés opposés (20, 21) entre lesdites extrémités de tête et de base (14, 15), lesdits bords latéraux (20, 21) étant incurvés de manière à produire une partie étranglée (19) de ladite plaque (10) entre lesdites extrémités de tête et de base (14, 15), dans lequel ladite partie étranglée (19) présente une largeur inférieure à la largeur de ladite extrémité de base (15) ; et
→ ladite plaque (10) définissant en outre une paire de trous (25) au niveau de chacune de ladite extrémité de tête (14) et de ladite extrémité de base (15) et une pluralité de trous (25) entre lesdites paires de trous (25) ;
dans lequel l'un desdits bords latéraux (20, 21) est incurvé selon un premier rayon et celui opposé desdits bords latéraux (20, 21) est incurvé selon un deuxième rayon qui est plus grand que ledit premier rayon.

2. Dispositif de fixation selon la revendication 1, dans lequel ladite pluralité de trous (25) sont décalés les uns par rapport aux autres.

3. Dispositif de fixation selon la revendication 2, dans lequel ladite pluralité de trous (25) comprend un premier groupe de trous (25) adjacent à l'un desdits bords latéraux (20, 21) et un deuxième groupe de trous (25) adjacent à celui opposé desdits bords latéraux (20, 21) et décalé par rapport audit premier groupe de trous (25).

4. Dispositif de fixation selon la revendication 3, dans lequel lesdits premier et deuxième groupes de trous (25) comprennent chacun au moins deux trous (25).

5. Dispositif de fixation selon la revendication 1, dans lequel ladite plaque allongée (10) est incurvé entre lesdits bords latéraux (20, 21) de manière sensiblement uniforme le long de la longueur de ladite plaque (10).

6. Dispositif de fixation selon la revendication 1, dans lequel certains au moins desdits trous (25) comprennent un chanfrein circonférentiel (26) configuré de telle sorte que ledit trou (25) accepte des vis de dimensions différentes.

7. Dispositif de fixation selon la revendication 1, dans lequel ladite plaque (10) présente une épaisseur qui est inférieure à 1 mm environ.
